# EUROPEAN PATENT SPECIFICATION

(11) **EP 2 352 558 B1**
(45) Date of publication and mention of the grant of the patent: **02.07.2014**
(21) Application number: 09741431.2
(22) Date of filing: 12.10.2009
(51) Int. Cl.: A61N 7/02, G06F 19/00

(54) **SYSTEM FOR ULTRASOUND THERAPY**
SYSTEM ZUR ULTRASCHALLTHERAPIE
SYSTÈME DE THÉRAPIE PAR ULTRASONS

(30) Priority: 04.11.2008 US 111002 P
(43) Date of publication of application: 10.08.2011
(73) Proprietor: Koninklijke Philips N.V., 5656 AE Eindhoven (NL)
(72) Inventor: DALAL, Sandeep, Briarcliff Manor, New York 10510-8001 (US); RAJU, Balasundara, Briarcliff Manor, New York 10510-8001 (US); ANAND, Ajay, Briarcliff Manor, New York 10510-8001 (US)
(74) Representative: van Velzen, Maaike Mathilde
(86) International application number: PCT/IB2009/054483
(87) International publication number: WO 2010/052596

(56) References cited:
- WO-A-03/076003
- WO-A-2007/014026
- WO-A-2007/123913
- US-A- 5 553 618
- US-A1- 2006 293 583
- US-A1- 2007 010 805
- US-A1- 2007 016 039
- US-A1- 2007 076 846
- US-A1- 2008 033 420

## Description

The present application relates to the therapeutic arts, in particular ultrasound ablation therapy and will be described with particular reference thereto. However, it is to be appreciated that the exemplary embodiments can also find application in conjunction with other therapeutic treatments and the like.

Ultrasound techniques are becoming a desirable approach for therapeutic interventions due in part to their non-invasive nature. The use of ultrasound in these applications allows non-invasive treatment of deep tissues. For example, the use of high-intensity focused ultrasound is being used by users for thermal therapeutic intervention of uterine fibroids. Ultrasound therapy for tissue ablation includes insonifying a tissue of interest with high intensity ultrasound that is absorbed and converted into heat, raising the temperature of the tissues. As the temperature rises above 55°C, coagulative necrosis of the tissues occurs resulting in immediate cell death. In this context, reference is made to US 2007/0016039 A1 and US 5,553,618.

In ultrasound-based tissue ablation, the ultrasound lesions used for tumor ablation are typically cigar-shaped, with a length along the ultrasound propagation direction and a width along the transverse direction. When electronically steered arrays are used to rapidly re-position the ultrasound beams, these ultrasound lesions can vary with the spatial location of the beam itself. For instance, when the ultrasound beam is made to focus on a tissue that is deep from the transducer, the beam sizes are typically much larger than the case when the beams are steered closer to the transducer. Also, when the ultrasound beams are steered off-axis, they tend to be larger and more elongated than the case where they are focused along the propagation axis. Typical dimensions of the ultrasound beam are 1 to 2 cm along propagation axis, and 1-2 mm in the transverse directions. The ultrasound lesion sizes depend not only on the ultrasound beam characteristics, but also on the sonication time.

In order to optimally treat tumors that are larger than the size of a single ultrasound lesion, the ultrasound beam needs to be repositioned in different parts of the tumor. This process is repeated until the entire tumor is covered with multiple ultrasound lesions or ablations. In practice, doctors treat an additional small margin encompassing the tumor to also treat microscopic cancerous tissue that may not be visible in the diagnostic imaging scans of the tumor. The tumor and margin is also knows as the Planned Target Volume (PTV). In contemporary systems, the placement of these ultrasound lesions one next to the other to achieve a three dimensional treatment volume is done in a non-optimal manner. If the PTV is larger than one ablation, which is frequently the case, a sequence of multiple overlapping ablations, called a "composite ablation", has to be created. Even if the PTV is only slightly larger than an individual ablation, a large number of ablations may be required, making the task of planning the composite ablation difficult to perform, especially in three dimensions. An example of a planning tool for computing a composite ablation to cover an arbitrary PTV with multiple overlapping ablations is described in patent publication WO/2008/09048. Although the publication describes a coverage algorithm for radiofrequency ablation planning, it can be used to compute the number and placement of ultrasound ablations. The planning tool uses a typical ablation shape to cover the PTV.

Contemporary devices using ultrasound ablation, such as the Sonablate® or Ablatherm® devices for prostate treatment, employ a mechanically focused transducer that has the same beam shape everywhere the focus is moved and employs straightforward placement of ablation lesions next to one another. However, dead spots between the ultrasound lesions or an unnecessarily large number of lesions with significant overlap in between are of concern.

Additionally, the actual observed ablation lesions can vary depending on many factors such as the local perfusion levels (e.g., presence of a major blood vessel nearby), the local ultrasound absorption rates, and the unknown intervening tissue attenuation. Since these are unknown a priori, the previously pre-planned treatment protocol is often inadequate and the actual lesions are smaller or larger than the planned ablation shapes. The procedure could use an unnecessarily large number of overlapping lesions if the actual lesions are larger than expected. On the other hand if the lesions were smaller than expected, the treatment could leave unablated gaps in the treatment volume.

Contemporary implementations of ultrasound ablation therapy try to cover a tumor volume by systematically exposing adjacent regions of tissue to thermal energy. One method can be by applying energy for longer durations of time at one location and diffusing the heat outwards from that point such that it covers the entire volume. Another method can be by scanning the region in a systematic manner by sequentially ablating small regions of tissue and moving the focus spot in a geometric scanning pattern to cover the entire volume. Neither of these contemporary techniques provides optimum locations for those ablations in terms of either improving the coverage of a planned target volume that contains the diseased region of tissue, minimizing damage to healthy tissue, or minimizing the total procedure time. All of these factors can be important based on the patient's prognosis and available procedure time.

The invention is defined in the appended claims.

A method disclosed herein can include obtaining first imaging of a region of interest; delineating a planned target volume using the first imaging; selecting ultrasound ablation shapes from a library of ultrasound ablation shapes; performing first ultrasound ablation on the planned target volume using the selected ultrasound ablation shapes; obtaining second imaging of the region of interest after performing the first ultrasound ablation; and adjusting the selected ultrasound ablation shapes, based at least in part on the second imaging.

The invention provides a computer-readable storage medium which includes a computer-executable code stored therein, wherein the computer-executable code is configured to cause a computing device in which the computer-readable storage medium is loaded to execute the steps of obtaining first imaging of a region of interest; receiving a selection of a planned target volume using the first imaging; selecting ultrasound ablation shapes from a candidate library of available ultrasound ablation shapes, wherein an ultrasound ablation shape of the library corresponds to a lesion shape at a respective focal point, and wherein ultrasound ablation shapes of the library have been pre-computed by a thermal acoustic model tool and/or obtained through experiments; performing first ultrasound ablation on the planned target volume using an ultrasound transducer that applies energy based on the selected ultrasound ablation shapes; obtaining second imaging of the region of interest after performing the first ultrasound ablation; selecting other ultrasound ablation shapes from the library of ultrasound ablation shapes based at least in part on the second imaging, wherein the other ultrasound ablation shapes are selected and modified by comparing observed ablation lesions from the second imaging with estimated ablation lesions; and performing second ultrasound ablation based at least in part on the selected and modified other ultrasound ablation shapes.

Furthermore, the invention provides an ultrasound ablation system which includes an imaging device for capturing first imaging of a region of interest, a processor for receiving a selection of a planned target volume using the first imaging, and an ultrasound transducer for performing first ultrasound ablation on the planned target volume. The processor is configured to select ultrasound ablation shapes from a candidate library of available ultrasound ablation shapes, wherein an ultrasound ablation shape of the library corresponds to a lesion shape at a respective focal point, and wherein the ultrasound ablation shapes of the library have been pre-computed by a thermal acoustic model tool and/or obtained through experiments. The ultrasound transducer can apply energy to the planned target volume based at least in part on the selected ultrasound ablation shapes. The imaging device can capture second imaging of the region of interest after application of the first ultrasound ablation. The processor is configured to select other ultrasound ablation shapes from the library of ultrasound ablation shapes based at least in part on the second imaging, wherein the other ultrasound ablation shapes are selected and modified by comparing observed ablation lesions from the second imaging with estimated ablation lesions.

In one embodiment, the processor can include a planning tool to plan the coverage of a tumor with overlapping ablations. The planning tool can use the library of available ablation shapes to cover the PTV. It can optimize one or more clinical objectives, such as guaranteeing the coverage of the entire PTV, minimizing the number of ablations, minimizing the ablation of healthy tissue, avoiding critical tissue, minimizing total treatment time, and so forth. The result of the planning tool can be a treatment plan. The plan can be accepted for continuation of the treatment after evaluation by the doctor. The planning tool can rapidly recalculate treatment plans as necessary enabling the doctor to re-weight objectives and/or modify physical parameters of the executed treatment. The planning tool can also visualize the treatment plan in the form of a graphical interface depicting the focal points for energy delivery and the ultrasound lesion shapes at those points as determined from the library. The exemplary embodiments used for the ultrasound-based tissue ablation can involve a combination of utilizing imaging information, the physical parameters of ablation therapy and positional feedback at the time of treatment.

The exemplary embodiments described herein can have a number of advantages over contemporary systems and processes, including reducing or eliminating dead spots between the ultrasound lesions and/or eliminating any unnecessarily large number of lesions with significant overlap. The exemplary embodiments described herein can provide for an efficient ultrasound ablation treatment of tissue, through reduced time and reduced ultrasound exposure.

The above-described and other features and advantages of the present disclosure will be appreciated and understood by those skilled in the art from the following detailed description, drawings, and appended claims.
Figure 1 is a schematic illustration of an exemplary embodiment of a system for use in ultrasonic ablation;
Figure 2 depicts a spatial map illustrating the layout of ablations created with a phased ultrasonic array;
Figure 3 depicts imaging with an applied treatment plan according to an embodiment of the present invention;
Figure 4 depicts imaging with another applied treatment plan according to an embodiment of the present invention; and
Figure 5 is a method that can be used by the system of FIG. 1 for ultrasound ablation.

The exemplary embodiments described herein provide a system of providing ultrasound ablation therapy in a PTV. The system and method has a number of advantages including ensuring more effective treatment through calculation of more accurate treatment volumes; ensuring adequate coverage and ensuring that critical treatment regions (e.g., tumors) are not missed; reducing damage to healthy tissues since an optimal number of lesion locations can be used rather than a larger number, which can cause unnecessary damage to healthy tissues such as the skin; minimizing treatment duration (e.g., if perfusion is low then the treatment can be made faster for that local region by reducing the sonication time). The exemplary embodiments of the present disclosure can include either or both of a feedback tool for updating intra-procedurally the placement of subsequent ablation volumes in the tissue and updating treatment protocol for subsequent locations.

The exemplary embodiments can provide a planning tool that allows a new treatment plan to be evaluated by the user, including users, physicians, operators, technicians or other individuals, prior to continuing the procedure and ensuring rapid calculation of treatment plans, which allows for re-weighting of objectives to calculate any modifications. The planning and feedback tool can include methods to visualize the treatment plan in terms of focal points for energy delivery and expected tissue damage surrounding the focal points individually or aggregated over all ablations.

In one embodiment, the system of the present disclosure can include an imaging device to acquire anatomical information from a patient (e.g. CT, ultrasound or MRI images); a system for user-based graphical delineation of one or more PTV's; and/or a library of tissue, transducer, and/or excitation dependent ablation shapes. The ablation shapes are pre-computed by a thermal acoustic modeling tool and/or obtained through experiments. The library may be stored in a memory or storage device.

In another embodiment, the system of the present disclosure can include a system for user-based interaction that allows the user to select a set of ablation shapes from the library of ablation shapes; a planning tool that calculates a set of ablations that satisfies a set of previously-stated objectives; a system for user-based weighting of the objectives used by the planning tool; a visualization device for visualizing the treatment plan in terms of focal points for energy delivery and expected tissue damage surrounding the focal points individually or aggregated over all ablations; an intra-procedural system to monitor ablation volumes (e.g., through imaging means such as MRI or ultrasound elastography); a feedback module that communicates the observed ablation shapes to the planning module; and/or a re-calculation module for recalculating and updating the set of ablation locations and shapes for ablating the remaining (e.g. untreated) portion of the treatment volume. For instance, the re-calculation module can be implemented as part of a planning module, as a specific mode of operation.

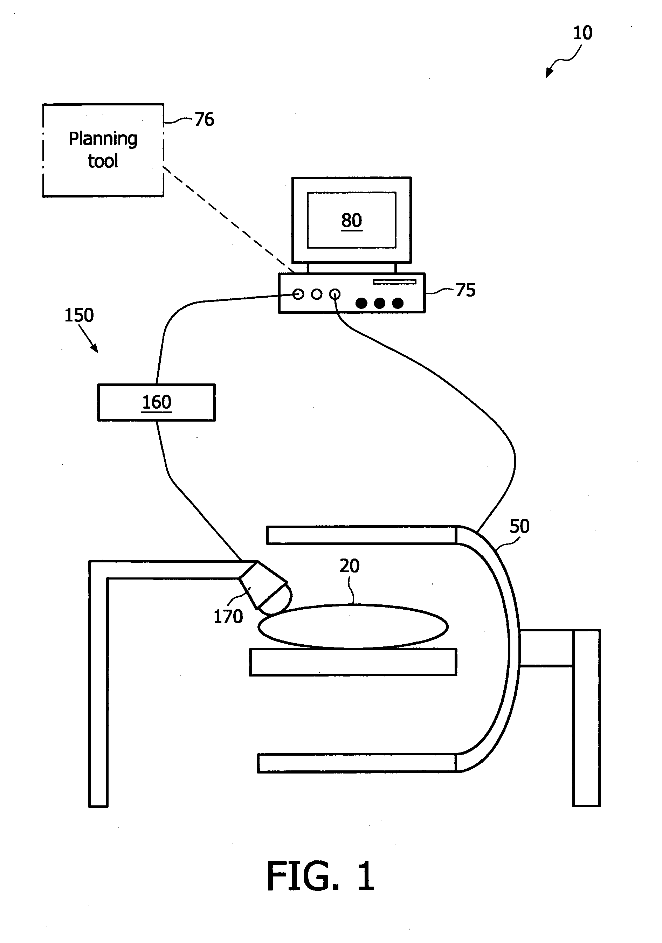

Referring to the drawings, and in particular to FIGS. 1-3, an ultrasound ablation system 10 can have an imaging device or system 50 and an ultrasound device or system 150 that can be utilized for ablation therapy in a patient 20. The particular type of ablation therapy can vary, including treatment of uterine fibroids, as well as liver, brain, prostate, and other cancerous lesions.

Each of the systems 50 and 150 can be in communication with a processor 75 having a display device 80 (e.g., a monitor) connected thereto. However, while the exemplary embodiment describes the systems 50 and 150 being connected to the processor 75, it would be understood by one of ordinary skill in the art that certain techniques that are described with respect to system 10 can be performed independently of other techniques. For example, the imaging system 50 can be an independent system that is utilized for obtaining images of the patient 20 prior to commencing the procedure while another imaging system can be utilized for providing imaging feedback as will be described again later.

In one embodiment, imaging system 50 can utilize MRI imaging, such as the system depicted in FIG. 1 using an open MRI device. System 50 can provide the user with images of the target region that can be delineated or otherwise marked to establish the PTV. The particular modality of the images can vary, such as ultrasound and CT modalities, and can also include combinations of modalities so that the user has a better depiction of the treatment area.

In one embodiment, the system 50 can acquire the images, display them on monitor 80 using the processor 75, and then provide a user interface for delineating the PTV. In another embodiment, the processor 75 can import the images of the patient and provide for delineation with graphical tools that can create and adjust (e.g. move, rotate, scale and so forth) the three-dimensional outline of the diseased tissue. In one embodiment, each region can be identified and classified as a PTV. In another embodiment, the user can outline critical structures that are near the PTV which are not intended to be damaged by the ultrasonic therapy system. The PTV and critical regions can be recorded for use by a planning tool 76 of the processor 75.

The ultrasound system 150 includes an ultrasound controller 160 and an ultrasound transducer or probe 170. The particular type of the ultrasound controller 160, the transducer 170 and other ultrasound components that are utilized by the system 10 can vary, and the particular ultrasound techniques can also vary. While the exemplary embodiments depict an external transducer 170 for delivering ultrasound energy to the PTV, the present disclosure contemplates the transducer being an internal transducer that delivers ultrasound energy to tissue positioned internally or in hard to reach places. The transducer 170 can have a size and shape that allows it to be inserted into the patient's body to the PTV, such as through a cannula, and can include being positioned through vessels, urethra, rectum, and so forth in the patient.

In one embodiment, the controller 160 can include components and/or utilize techniques associated with steering and electronic focusing of the ultrasound waves of the transducer 170. The present disclosure also contemplates the use of other components and/or techniques in addition to, or in place of, the components of the controller 160 described above. It should further be understood by one of ordinary skill in the art that the controller 160 or one or more of its components can be incorporated into, or shared with, the processor 75, such as for data processing and presentation techniques.

The transducer 170 can include an array of transducer or acoustic elements for the transmission of ultrasonic waves and for the receipt of ultrasonic echo signals. In one embodiment, the transducer 170 can provide for steering and electronic focusing of the ultrasound waves with respect to the target region to be treated. By appropriately delaying the pulses applied to each transducer element, the transducer 170 can transmit a focused ultrasound beam along a desired transmit scan line. According to one embodiment, the array transducer 170 can include a two-dimensional array such as disclosed in U.S. Pat. No. 6,428,477. In one embodiment, the system 10 can deliver ultrasound therapy beams that are highly focused, e.g. high-intensity focused ultrasound (HIFU). However, the present disclosure also contemplates other ultrasound techniques, including less-focused beams, for performing the tissue ablation.

The planning tool 76 of the processor 75 can create a set of ablation shapes that are related to the tissue characteristics, the transducer's physical characteristics, and/or parameters chosen for the specific application of energy delivery through the transducer 170. The tissue characteristics can modulate the actual size and shape of the ablation lesions. The transducer's physical characteristics can include the number, shapes and location of the various basic elements that are actively used in the transducer 170, which influence the focal-point of an ablation lesion, and its corresponding shape. The energy delivery parameters can include the power applied and the exposure time at any given location. The combination of these transducer and tissue characteristics, and energy delivery parameters when used within an appropriately designed thermal acoustic modeling tool can provide a method to derive a library of ablation shapes that can be used by the planning tool 76.

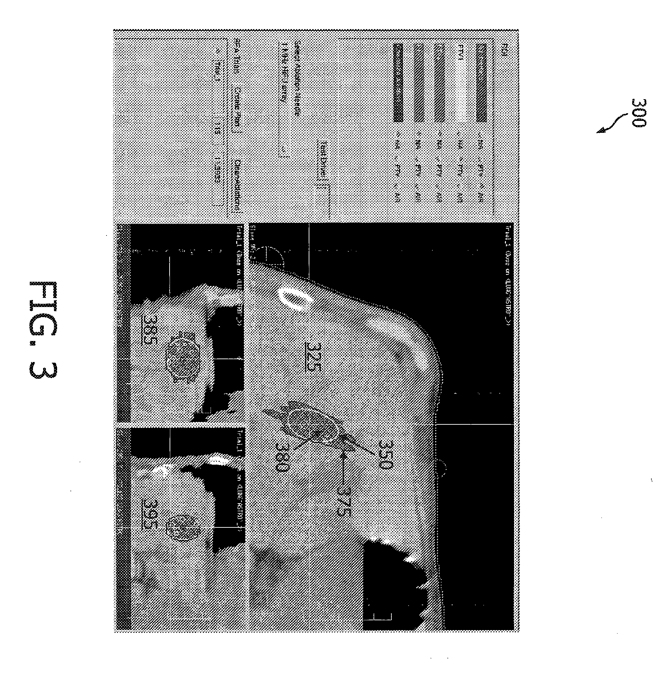

The planning tool 76 can choose between ablations of different shapes and sizes that best match one or more treatment objectives defined by the user. If the user has a weighting of the objectives into the processor 75, the planning tool 76 can adapt its results according to the weighting. A resulting treatment plan can be visualized graphically as shown for example in FIG. 3.

The library of tissue, transducer, and excitation dependent ablation shapes can be created by a thermal acoustic modeling tool that utilizes knowledge of organ or tissue-type, the expected transducer characteristics and the parameters used for delivering the acoustical energy. The calculation of an ablation shape can be performed at a specific location in 3-D space with respect to the transducer's position. For instance, Table 1 shows an example of acoustic beam widths along the axial, transverse, and elevation directions for different ultrasonic array configurations:

**Table 1:**

| | | | ***Acoustic* Beam Widths** | | |
|---|---|---|---|---|---|
| **Frequency & Aperture Size** | | ***Focus Location*** | ***Axial*** | ***Transverse*** | ***Elevation*** |
| | | ***(mm)*** | ***(mm)*** | ***(mm)*** | ***(mm)*** |
| ***1.50 Linear Arrays*** | | | | | |
| | | | | | |
| *1 MHz* | | [0 0 60] | 24.24 | 2.41 | 4.70 |
| (2.5 cm Elevation: 5 cm Lateral) | | [25 0 45] | 24.73 | 3.08 | 4.05 |
| | | | | | |
| *2 MHz* | | [0 0 60] | 12.50 | 1.21 | 4.71 |
| (1.25 cm Elevation: 5 cm Lateral) | | [25 0 45] | 11.47 | 1.42 | 4.04 |
| | | | | | |
| *4 MHz* | | [0 0 60] | 6.30 | 0.63 | |
| *(0.6 cm Elevation 5 cm Lateral)* | | [25 0 45] | 6.30 | 0.62 | |
| | | | | | |
| ***2D Random Arrays*** | | | | | |
| | | | | | |
| *1 MHz* | | [0 0 60] | 24.25 | 2.57 | 4.55 |
| *(2.5 cm Elevation: 5 cm Lateral)* | | [25 0 45] | 24.30 | 2.69 | 4.40 |

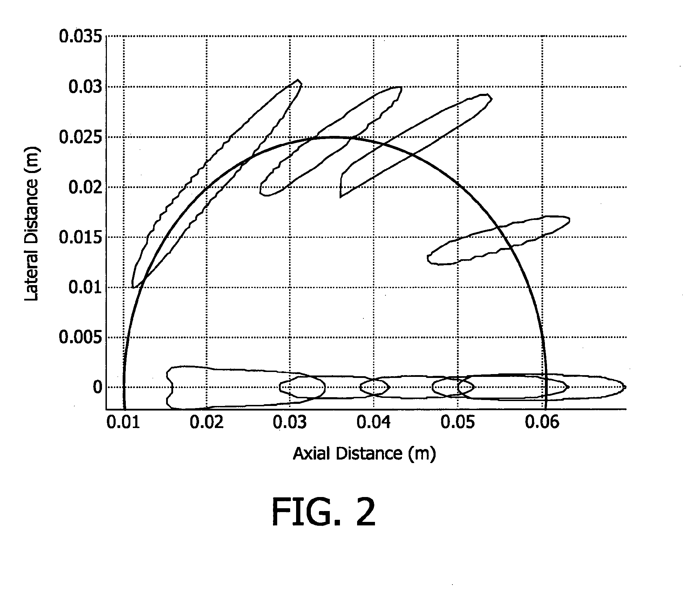

The acoustic beam width can be used to influence the ablation shape that results from using the acoustic beam with certain energy delivery parameters, e.g. power and time duration. Figure 2 shows actual ablation shapes for a given transducer when used at a specified focus location (e.g. indexed by axial and lateral distances from the transducer tip) for a specified amount of time as shown in Table 2:

**Table 2:**

| **Axial Distance (cm)** | **Lateral Distance (cm)** | **Lesion Volume (cc)** | **Time (sec)** |
|---|---|---|---|
| 2.5 | 0.00 | 0.1669 | 20 |
| 2.5 | 2.29 | 0.4 | 15.5 |
| 3.5 | 0.00 | 0.037 | 3 |
| 3.5 | 2.50 | 0.047 | 2.5 |
| 4.5 | 0.00 | 0.039 | 2.5 |
| 4.5 | 2.29 | 0.08 | 3 |
| 5.5 | 0.00 | 0.0602 | 3 |
| 5.5 | 1.50 | 0.067 | 3 |
| 6 | 0.00 | 0.099 | 4 |
| | **Total** | **0.9961** | **56.5** |

The planning tool 76 of the processor 75 can combine an algorithm that covers the entire PTV with set of ablation shapes in which the shapes are restricted to be as defined in the library. The planning tool 76 can operate in conjunction with the set of user's objectives, such as minimizing the number of ablations over and above any consideration of total treatment time, or ablating more healthy tissue at the cost of total treatment time. In one embodiment, the user can select from a list of weighting of objectives through a text or a graphical based interface to the planning tool 76.

Display device 80 can provide for a visualization of the treatment plan by depicting the ablation shapes overlaid on the PTV regions. The graphical depiction can include the focal points for energy delivery and expected shapes of the ablation surrounding the focal points either individually or aggregated over all ablations. This graphical depiction 300 is shown in FIG. 3, and includes the tissue in the region of interest 325, the PTV 350, the composite ablation 375, individual energy delivery focal points 380 and corresponding ablation shapes, and additional imaging slices in other imaging planes 385, 395. The composite ablation 375 can be composed with multiple ablation shapes, in this figure it includes only one shape of size 2cm × 0.25cm × 0.25cm. The graphical depiction 300 shows the total number of ablations as 115 and collateral damage as 11.4 cc of healthy tissue.

In one embodiment, the user can limit the planning tool 76 to use a limited set of ablation shapes from the library. This limit can reduce the processing time of the planning tool. In one embodiment, the planning tool 76 can notify the user of how the transducer is most efficiently used. In another embodiment, the planning tool 76 can account for constraints including the sequence of ablations given that distal ablations if performed early on in the procedure may not impact proximal ablations. This can reduce the complexity of the planning algorithm in that it reduces the search space of possible candidate ablation locations.

In another embodiment, the processor 75 can perform ablations with dead time for cooling to prevent damage to critical structures. The total time for the procedure can take this dead time into account. The planning tool 76 can adapt the sequence of ablations in a manner to cover separate PTVs to overlap the dead time for one region of interest with the active time for another. Even in the case of one region of interest, the exemplary embodiments can sequentially switch between sub-apertures of an ultrasonic array to ablate substantially different regions of the tissue in order to prevent damage to surface structures.

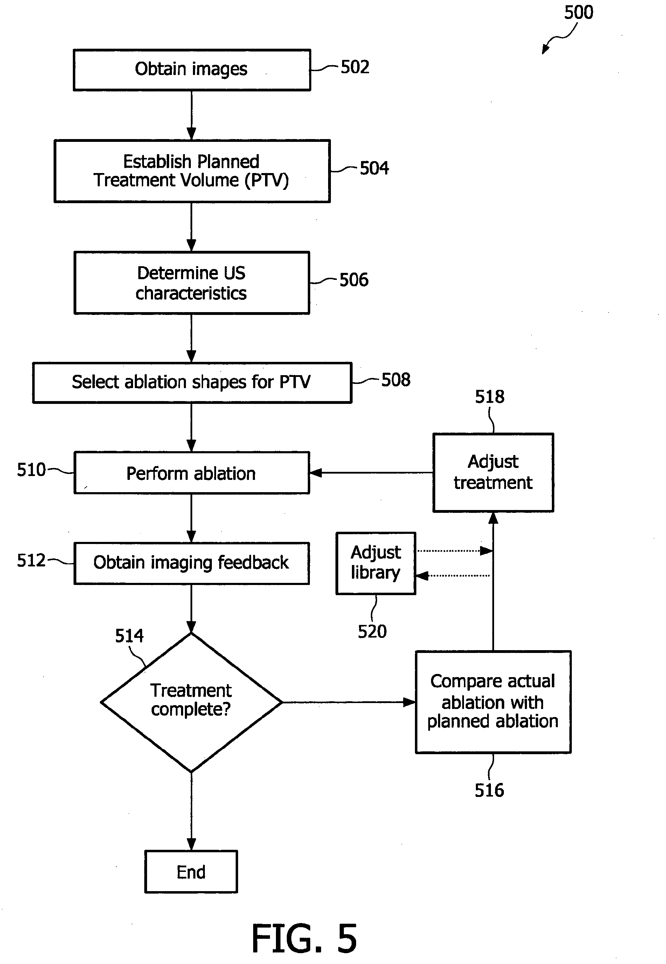

Referring additionally to FIG. 5, a method that may be utilized for performing ultrasound ablation therapy with the system 10 is shown and generally represented by a reference numeral 500. It should be understood by one of ordinary skill in the art that the steps described with respect to the method 500 are intended to be exemplary of the use of the system 10, and more or less steps can be employed. Additionally, other components or devices that are not specifically described with respect to the system 10 can also be employed in the performance of the method 500. The method 500 can commence with the step 502 where images of a region of interest of a patient are obtained by the processor 75. The region of interest can include one or more tumors or other treatment areas for the patient. In step 504, a user can describe or otherwise establish one or more planned target volumes (PTVs) based on the pre-procedural image data sets.

In step 506, the processor 75 can obtain characteristics associated with the ultrasound transducer, such as power, transducer acoustic frequency, and f-number of the transducer. In step 508, the processor 75 can select the ablation shaped from the library. For instance, a thermal acoustic modeling module can provide candidate ablation shapes based on the power, the transducer acoustic frequency, the f-number of transducer, and the duration of treatment for different spatial locations within the PTV, which are selected from the library of ablation shapes. In one embodiment, the ablation shapes can be location specific. The planning tool 76 of the processor 75 can use the library of candidate ablations to pre-select a set of ablation shapes that completely cover the PTV without destroying critical structures that are healthy.

In step 510, the ablation therapy can be initiated by applying the ultrasound energy to the tissue. In step 512, the processor 75 can perform intra-operative ablation shape monitoring through imaging feedback, e.g., MRI temperature mapping. While the exemplary embodiment describes MRI temperature measurements being utilized as the feedback mechanism, other imaging modalities can also be used, such as elastography techniques using ultrasound.

In one embodiment, the feedback can be obtained frequently, such as every 3 to 5 seconds,where MRI imaging is being used for the feedback. The frequency of the feedback can be based on a number of factors including the type of imaging being used for feedback. The imaging feedback can provide a spatial map of what has been ablated so far during the procedure. The method 500 can determine if the ablation therapy has been completed in step 514 and, if not, then it can proceed to step 516 where the processor 75 can compare the actual ablated shapes and volumes with the pre-treatment estimates.

In step 518, the processor 75 can apply the feedback data for adjustment of the ablation therapy. For example, if the observed volumes are larger than the pre-determined estimates (e.g., there is less perfusion which tends to increase the temperature rise in that location), then the remaining ablation shapes for the remainder of the tissue (at least locally close to that lesion) can be chosen to be larger with fewer ablation points. This can be accomplished automatically and/or can be verified by the user before it takes effect. On the other hand, if the observed ablation volumes are smaller than the pre-determined estimates, then the remaining ablation shapes can be chosen to be smaller in order to reduce or eliminate dead-spots in between.

In one embodiment, the planning tool 76 using the feedback data can establish the degree of conformance of the observed ablation shape with the expected shape from the pre-procedural plan. The planning tool 76 can perform a re-calculation if the remaining pre-procedurally planned ablation locations and shapes would create a complete plan satisfying all the weighted objectives for the remaining tumor volume; and if not, it can re-calculate a new plan for the remaining tumor volume.

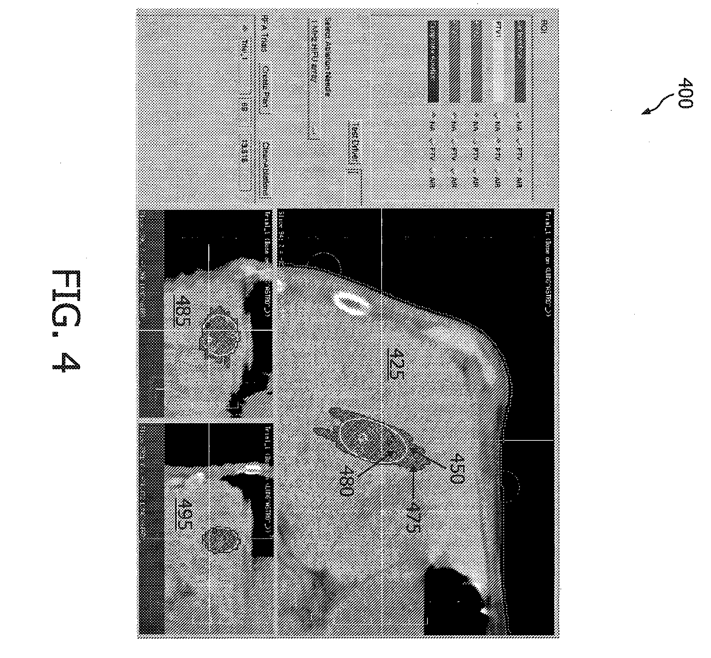

In one embodiment in step 520, the candidate library of ablation shapes can be updated based on the feedback data. The library, or a portion thereof, that has ablation shapes updated based on the feedback data can be made patient specific. For example as shown in FIG. 4, the treatment of a pre-determined volume of interest with lesion sizes of 2 cm x 0.35 cm x 0.35 cm is shown, which was selected based on nominal tissue parameters. This graphical depiction 400 shown in FIG. 4 includes the tissue 425, the PTV 450, the composite ablation 475, individual focal points for energy delivery 480 and corresponding ablation shapes, and additional imaging slices in other imaging planes 485, 495. The graphical depiction 400 shows treatment when a patient's perfusion is lower than expected leading to larger lesions so that only 69 lesions are needed to cover the PTV. In this instance, the processor 75 can utilize the captured feedback data capture to determine that the actual observed lesion size is larger than the pre-planned one, it would update the ablation size/shape in the library to reflect this information, the planning tool 76 would use the updated ablation shape from the library, and then re-calculate the plan with only 69 ablation lesions to cover the treatment volume. In this example, if the original treatment plan had been continued without the benefit of intra-procedural feedback, it would have resulted in a 67% longer treatment time and also would have lead to a larger ultrasound dose to intervening tissues such as the skin surface. In practice, the perfusion and other properties could locally vary and certain sub-regions could use the smaller lesions and other sub-regions could use the larger lesion volumes. This treatment procedure can provide for full treatment with reduced treatment time.

In another embodiment, the system 10 can have a calibration step where ablation lesions are placed at a sufficient number of points in the PTV so as to sufficiently sample the lesion formation behavior throughout the PTV. In one embodiment, these ablations can be performed in sequence. The observed lesions can then be compared to the pre-planned lesions to update the library of ablation size/shapes, and the planning tool 76 can re-plan the treatment protocol taking into account the updated ablation shapes at the different sampling locations. The treatment could then be initiated and would require less feedback during the actual procedure after the calibration step is completed.

The exemplary embodiments provide for a pre-procedural calculation and graphical visualization of all ablation locations for execution of an ultrasonic therapy treatment. The plan allows for ablations to be created in a range of sizes and shapes based upon parameters of energy delivery. The planning tool 76 can utilize knowledge of the complete range of sizes and shapes that are described in the library of ablations. This library can be created in a number of ways, including an acoustic modeling tool that uses knowledge of organ or tissue type, as well as the expected transducer characteristics, and/or it can be created through experimental means.

The exemplary embodiments also provide for the ability to create an treatment plan that can satisfy one or more of the following objectives: complete ablation of the PTV; minimizing the number of ablations; minimizing the thermal dose to the healthy tissue surrounding the PTV; maintaining safety (e.g. no ablation) for any identified critical structures; minimizing total procedure time; using a fixed set of ablation shapes from the library; and/or selecting a sequence of ablations that minimize thermal injury in the near-field. Treatment objectives can be weighted by the doctor prior to the procedure. In one embodiment, the present disclosure provides for ensuring rapid calculation of treatment plans, which allows for re-weighting of objectives to calculate any modifications.

The exemplary embodiments of the present disclosure are described with respect to ultrasound ablation therapy to treat tumors in a human. It should be understood by one of ordinary skill in the art that the exemplary embodiments of the present disclosure can be applied to other types of medical intervention and other portions of the body, whether human or animal, including for such other treatments as mediating clot dissolution (sonothrombolysis), mediating drug delivery and gene therapy, cosmetic means (e.g., removal of fat).

For example, the methods and systems described with respect to the exemplary embodiments can be used to increase the efficacy of existing medical treatments such as the use of tPA for stroke patients. The exemplary embodiments can be used with ultrasound mediated drug delivery and gene therapy. Genetic expression of proteins in gene therapy, and increased delivery of drugs in site-targeted therapies have potential to treat a wide variety of diseases with minimal side-effects. The exemplary embodiments described herein provide for the use of ultrasound for non-invasive treatment of deep tissues with little or no effect on overlying organs. The exemplary embodiments can also be applied to non-humans, such as in pre-clinical animal research. In one embodiment, microbubble and/or nanoparticle ultrasound contrast agents can be used for imaging, targeting and delivery of therapeutic payloads as described with respect to the exemplary embodiments. The present disclosure also contemplates use of other energy delivery methods, including radio frequency ablation.

The invention, including the steps of the methodologies described above, can be realized in hardware, software, or a combination of hardware and software. The invention can be realized in a centralized fashion in one computer system, or in a distributed fashion where different elements are spread across several interconnected computer systems. Any kind of computer system or other apparatus adapted for carrying out the methods described herein is suited. A typical combination of hardware and software can be a general purpose computer system with a computer program that, when being loaded and executed, controls the computer system such that it carries out the methods described herein.

The invention, including the steps of the methodologies described above, can be embedded in a computer program product. The computer program product can comprise a computer-readable storage medium in which is embedded a computer program comprising computer-executable code for directing a computing device or computer-based system to perform the various procedures, processes and methods described herein. Computer program in the present context means any expression, in any language, code or notation, of a set of instructions intended to cause a system having an information processing capability to perform a particular function either directly or after either or both of the following: a) conversion to another language, code or notation; b) reproduction in a different material form.

The illustrations of embodiments described herein are intended to provide a general understanding of the structure of various embodiments, and they are not intended to serve as a complete description of all the elements and features of apparatus and systems that might make use of the structures described herein. Many other embodiments will be apparent to those of skill in the art upon reviewing the above description. Other embodiments may be utilized and derived therefrom, such that structural and logical substitutions and changes may be made without departing from the scope of this disclosure. Figures are also merely representational and may not be drawn to scale. Certain proportions thereof may be exaggerated, while others may be minimized. Accordingly, the specification and drawings are to be regarded in an illustrative rather than a restrictive sense.

Thus, although specific embodiments have been illustrated and described herein, it should be appreciated that any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description. Therefore, it is intended that the disclosure not be limited to the particular embodiment(s) disclosed as the best mode contemplated for carrying out this invention, but that the invention will include all embodiments falling within the scope of the appended claims.

## Claims

1. A computer-readable storage medium in which a computer-executable code is stored, the computer-executable code being configured to cause a system comprising ultrasound ablation means, imaging means, a processor, a library of available candidate ultrasound ablation shapes and means for loading the computer-readable storage medium to execute the steps of:
causing the imaging means to obtain first imaging of a region of interest;
causing the processor to enable a graphical delineation of a planned target volume using the first imaging;
causing the processor to select ultrasound ablation shapes from the library of available candidate ultrasound ablation shapes, wherein an ultrasound ablation shape of the library corresponds to a lesion shape at a respective focal point, and wherein ultrasound ablation shapes of the library have been pre-computed by a thermal acoustic model tool and/or obtained through experiments;
causing the ultrasound ablation means to perform a first ultrasound ablation on the planned target volume using an ultrasound transducer (170) that applies energy based on the selected ultrasound ablation shapes;
causing the imaging means to obtain second imaging of the region of interest after performing the first ultrasound ablation;
causing the processor to select other ultrasound ablation shapes from the library of ultrasound ablation shapes based at least in part on the second imaging, wherein the other ultrasound ablation shapes are selected and modified by comparing observed ablation lesions from the second imaging with estimated ablation lesions; and
causing the ultrasound ablation means to perform a second ultrasound ablation based at least in part on the selected and modified other ultrasound ablation shapes.

2. The computer-readable storage medium of claim 1, further comprising a computer-executable code for causing the system to display the first imaging, determine a treatment plan using selected ultrasound ablation shapes from the library, and display the treatment plan, wherein the treatment plan comprises a graphic representation of the planned target volume, energy delivery focal points, the ultrasound ablation shapes, and a composite ablation comprising multiple ablation shapes overlaid on the planned target volume within the region of interest.

3. The computer-readable storage medium of claim 1, further comprising a computer-executable code for causing the system to store the selected and modified other ultrasound ablation shapes in the library of ultrasound ablation shapes.

4. The computer-readable storage medium of claim 1, further comprising a computer-executable code for causing the system to receive treatment objectives inputted by a user; and select the ultrasound ablation shapes from the library of ultrasound ablation shapes based at least in part on the treatment objectives and ultrasound characteristics associated with the ultrasound transducer (170) that performs the first ultrasound ablation.

5. The computer-readable storage medium of claim 1, further comprising a computer-executable code for causing the system to use a group of the ultrasound ablation shapes from the library of ultrasound ablation shapes based at least in part on an ablation model and ultrasound characteristics associated with the ultrasound transducer (170) that performs the first ultrasound ablation.

6. An ultrasound ablation system comprising:
an imaging device (50) for capturing first imaging of a region of interest;
a processor (75) having a planning tool (76) configured to determine a treatment plan, the processor being configured to enable a graphical delineation of a planned target volume using the first imaging;
a library of available candidate ultrasound ablation shapes, wherein an ultrasound ablation shape of the library corresponds to a lesion shape at a respective focal point, and wherein the ultrasound ablation shapes of the library have been pre-computed by a thermal acoustic model tool and/or obtained through experiments;
and
an ultrasound transducer (170) configured to perform a first ultrasound ablation on the planned target volume,
wherein the processor is configured to select ultrasound ablation shapes from the library of ablation shapes,
and wherein the ultrasound transducer is configured to apply energy to the planned target volume based at least in part on the treatment plan including the selected ultrasound ablation shapes,
wherein the imaging device is configured to capture second imaging of the region of interest after application of the first ultrasound ablation,
wherein the processor is configured to select other ultrasound ablation shapes from the library of ultrasound ablation shapes based at least in part on the second imaging, and wherein the processor is configured to select and modify the other ultrasound ablation shapes by comparing observed ablation lesions from the second imaging with estimated ablation lesions.

7. The system of claim 6, wherein the ultrasound transducer (170) is configured to perform a second ultrasound ablation based at least in part on the selected other ultrasound ablation shapes.

8. The system of claim 7, further comprising a display device (80) for presenting the first imaging and a treatment plan, wherein the treatment plan comprises a graphic representation of the planned target volume, the energy delivery focal points, the ultrasound ablation shapes and a composite ablation comprising multiple ablation shapes overlaid on the planned target volume within the region of interest.

9. The system of claim 6, wherein the processor (75) is configured to receive treatment objectives entered by a user, and wherein the planning tool (76) is configured to use a set of the ultrasound ablation shapes from the library of ultrasound ablation shapes based at least in part on the treatment objectives and ultrasound characteristics associated with the ultrasound transducer (170) that performs the first ultrasound ablation.

10. The system of claim 6, wherein the processor (75) is configured to select and modify the other ultrasound ablation shapes by comparing observed ablation lesions from the second imaging with estimated ablation lesions, wherein the modification is based on at least one of shape, size, location and number of the ultrasound ablation shapes.

11. The system of claim 6, wherein the imaging device (50) is configured to perform at least one of the second imaging by capturing temperature measurements of the region of interest using magnetic resonance imaging; ultrasound imaging associated with a shape of the region of interest; and elastography.

12. The computer-readable storage medium of claim 1, further comprising computer-executable code for calculating, based on the degree of conformance of the observed ablation shape with the expected shape, a set of ablation locations associated with the calculated ultrasound ablation shapes for ablating the remaining portion of the treatment volume.

13. The system of claim of claim 6, wherein the processor is configured to calculate, based on the degree of conformance of the observed ablation shape with the expected shape, a set of ablation locations associated with the calculated ultrasound ablation shapes for ablating the remaining portion of the treatment volume.

## Patentansprüche

1. Durch einen Computer lesbares Speichermedium, in dem ein durch einen Computer ausführbarer Code gespeichert ist, wobei der durch einen Computer ausführbare Code konfiguriert ist, um ein System mit Ultraschallablationsmitteln, Bildgebungsmitteln, einem Prozessor, einer Bibliothek von verfügbaren, in Frage kommenden Ultraschallablationsformen und Mitteln zum Laden des durch einen Computer lesbaren Speichermediums zu veranlassen, die folgenden Schritte durchzuführen:
Veranlassen der Bildgebungsmittel, eine erste Abbildung einer interessierenden Region zu erlangen;
Veranlassen des Prozessor, eine graphische Abgrenzung eines geplanten Zielvolumens unter Verwendung der ersten Abbildung zu ermöglichen;
Veranlassen des Prozessors zum Auswählen von Ultraschallablationsformen aus der Bibliothek der verfügbaren, in Frage kommenden Ultraschallablationsformen, wobei eine Ultraschallablationsform der Bibliothek einer Läsionsform an einem jeweiligen Fokalpunkt entspricht, und wobei Ultraschallablationsformen der Bibliothek durch ein thermoakustisches Modell-Werkzeug vorberechnet wurden und/oder durch Versuche erlangt wurden;
Veranlassen der Ultraschallablationsmittel zum Durchführen einer ersten Ultraschallablation an dem geplanten Zielvolumen unter Verwendung eines Ultraschallwandlers (170), der Energie basierend auf den ausgewählten Ultraschallablationsformen abgibt;
Veranlassen der Bildgebungsmittel, eine zweite Abbildung der interessierenden Region zu erlangen, nachdem die erste Ultraschallablation durchgeführt wurde;
Veranlassen des Prozessors, weitere Ultraschallablationsformen aus der Bibliothek der Ultraschallablationsformen basierend zumindest teilweise auf der zweiten Abbildung auszuwählen, wobei die weiteren Ultraschallablationsformen ausgewählt und modifiziert werden, indem beobachtete Ablationsläsionen aus der zweiten Abbildung mit geschätzten Ablationsläsionen verglichen werden; und
Veranlassen der Ultraschallablationsmittel zum Durchführen einer zweiten Ultraschallablation basierend zumindest teilweise auf den ausgewählten und modifizierten Ultraschallablationsformen.

2. Durch einen Computer lesbares Speichermedium nach Anspruch 1, weiterhin umfassend einen durch einen Computer ausführbaren Code zum Veranlassen des Systems, die erste Abbildung anzuzeigen, einen Therapieplan unter Verwendung ausgewählter Ultraschallablationsformen aus der Bibliothek festzulegen und den Therapieplan anzuzeigen, wobei der Therapieplan eine graphische Darstellung des geplanten Zielvolumens, Fokalpunkte für die Energieabgabe, die Ultraschallablationsformen und eine zusammengesetzte Ablation mit mehreren Ablationsformen, die dem geplanten Zielvolumen innerhalb der interessierenden Region überlagert sind, umfasst.

3. Durch einen Computer lesbares Speichermedium nach Anspruch 1, weiterhin umfassend einen durch einen Computer ausführbaren Code zum Veranlassen des Systems, die ausgewählten und modifizierten weiteren Ultraschallablationsformen in der Bibliothek der Ultraschallablationsformen zu speichern.

4. Durch einen Computer lesbares Speichermedium nach Anspruch 1, weiterhin umfassend einen durch einen Computer ausführbaren Code zum Veranlassen des Systems, durch einen Benutzer eingegebene Therapiezielsetzungen zu empfangen; und die Ultraschallablationsformen aus der Bibliothek der Ultraschallablationsformen basierend zumindest teilweise auf den Behandlungszielsetzungen und den zu dem Ultraschallwandler (170), der die erste Ultraschallablation durchführt, gehörigen Ultraschalleigenschaften auszuwählen.

5. Durch einen Computer lesbares Speichermedium nach Anspruch 1, weiterhin umfassend einen durch einen Computer ausführbaren Code zum Veranlassen des Systems, eine Gruppe von Ultraschallablationsformen aus der Bibliothek der Ultraschallablationsformen basierend zumindest teilweise auf einem Ablationsmodell und den zu dem Ultraschallwandler (170), der die erste Ultraschallablation durchführt, gehörigen Ultraschalleigenschaften zu verwenden.

6. Ultraschallablationssystem, das Folgendes umfasst:
eine Bildgebungsvorrichtung (50) zum Erfassen einer ersten Abbildung einer interessierenden Region;
einen Prozessor (75) mit einem Planungswerkzeug (76), konfiguriert, um einen Therapieplan festzulegen, wobei der Prozessor konfiguriert ist, um eine graphische Abgrenzung eines geplanten Zielvolumens unter Verwendung der ersten Abbildung zu ermöglichen;
eine Bibliothek von verfügbaren, in Frage kommenden Ultraschallablationsformen, wobei eine Ultraschallablationsform der Bibliothek einer Läsionsform an einem jeweiligen Fokalpunkt entspricht, und wobei die Ultraschallablationsformen der Bibliothek durch ein thermoakustisches Modell-Werkzeug vorberechnet wurden und/oder durch Versuche erlangt wurden; und
einen Ultraschallwandler (170), der konfiguriert ist, um eine erste Ultraschallablation an dem geplanten Zielvolumen durchzuführen,
wobei der Prozessor konfiguriert ist, um Ultraschallablationsformen aus der Bibliothek der Ablationsformen auszuwählen, und wobei der Ultraschallwandler konfiguriert ist, um Energie an das geplante Zielvolumen basierend zumindest teilweise auf dem Therapieplan mit den ausgewählten Ultraschallablationsformen abzugeben,
wobei die Bildgebungsvorrichtung konfiguriert ist, um eine zweite Abbildung der interessierenden Region nach Anwendung der ersten Ultraschallablation zu erfassen, wobei der Prozessor konfiguriert ist, um weitere Ultraschallablationsformen aus der Bibliothek der Ultraschallablationsformen basierend zumindest teilweise auf der zweiten Abbildung auszuwählen, und wobei der Prozessor konfiguriert ist, um die weiteren Ultraschallablationsformen auszuwählen und zu modifizieren, indem er beobachtete Ablationsläsionen aus der zweiten Abbildung mit geschätzten Ablationsläsionen vergleicht.

7. System nach Anspruch 6, wobei der Ultraschallwandler (170) konfiguriert ist, um eine zweite Ultraschallablation basierend zumindest teilweise auf den ausgewählten weiteren Ultraschallablationsformen durchzuführen.

8. System nach Anspruch 7, weiterhin umfassend eine Anzeigevorrichtung (80) zum Darstellen der ersten Abbildung und eines Therapieplans, wobei der Therapieplan eine graphische Darstellung des geplanten Zielvolumens, Fokalpunkte für die Energieabgabe, die Ultraschallablationsformen und eine zusammengesetzte Ablation mit mehreren Ablationsformen, die dem geplanten Zielvolumen innerhalb der interessierenden Region überlagert sind, umfasst.

9. System nach Anspruch 6, wobei der Prozessor (75) konfiguriert ist, um durch einen Benutzer eingegebene Behandlungszielsetzungen zu empfangen, und wobei das Planungswerkzeug (76) konfiguriert ist, um eine Gruppe der Ultraschallablationsformen aus der Bibliothek der Ultraschallablationsformen basierend zumindest teilweise auf den Behandlungszielsetzungen und den zu dem Ultraschallwandler (170), der die erste Ultraschallablation durchführt, gehörigen Ultraschalleigenschaften zu verwenden.

10. System nach Anspruch 6, wobei der Prozessor (75) konfiguriert ist, um die weiteren Ultraschallablationsformen auszuwählen und zu modifizieren, indem er beobachtete Ablationsläsionen aus der zweiten Abbildung mit geschätzten Ablationsläsionen vergleicht, wobei die Modifikation auf zumindest entweder der Form, der Größe, dem Ort oder der Anzahl der Ultraschallablationsformen basiert.

11. System nach Anspruch 6, wobei die Bildgebungsvorrichtung (50) konfiguriert ist, um zumindest entweder die zweite Abbildung durch Erfassen von Temperaturmesswerten der interessierenden Region unter Verwendung von Magnetresonanzbildgebung, zu einer Form der interessierenden Region gehörige Ultraschallbildgebung oder Elastographie durchzuführen.

12. Durch einen Computer lesbares Speichermedium nach Anspruch 1, weiterhin umfassend einen durch einen Computer ausführbaren Code, um basierend auf dem Grad der Übereinstimmung der beobachteten Ablationsform mit der erwarteten Form eine Gruppe von Ablationsorten zu berechnen, die zu den berechneten Ultraschallablationsformen gehören, um den verbleibenden Teil des Behandlungsvolumens zu abladieren.

13. System nach Anspruch 6, wobei der Prozessor konfiguriert ist, um basierend auf dem Grad der Übereinstimmung der beobachteten Ablationsform mit der erwarteten Form eine Gruppe von Ablationsorten zu berechnen, die zu den berechneten Ultraschallablationsformen gehören, um den verbleibenden Teil des Behandlungsvolumens zu abladieren.

## Revendications

1. Support de stockage lisible par ordinateur dans lequel est stocké un code exécutable par ordinateur, le code exécutable par ordinateur étant configuré pour faire en sorte qu'un système, comprenant des moyens d'ablation par ultrasons, des moyens d'imagerie, un processeur, une bibliothèque de formes d'ablation par ultrasons candidates disponibles et des moyens pour charger le support de stockage lisible par ordinateur, exécute les étapes consistant à :
faire en sorte que les moyens d'imagerie obtiennent une première image d'une région d'intérêt ;
permettre au processeur d'effectuer une délimitation graphique d'un volume cible planifié au moyen de la première image ;
faire en sorte que le processeur sélectionne des formes d'ablation par ultrasons à partir de la bibliothèque de formes d'ablation par ultrasons candidates disponibles, une forme d'ablation par ultrasons de la bibliothèque correspondant à une forme de lésion à un point focal respectif, et les formes d'ablation par ultrasons de la bibliothèque ayant été pré-calculées par un outil de modèle acoustique thermique et/ou obtenues par le biais d'expériences ;
faire en sorte que les moyens d'ablation par ultrasons opèrent une première ablation par ultrasons sur le volume cible planifié au moyen d'un transducteur ultrasonore (170) qui applique de l'énergie sur la base des formes d'ablation par ultrasons sélectionnées ;
faire en sorte que les moyens d'imagerie obtiennent une seconde image de la région d'intérêt après réalisation de la première ablation par ultrasons ;
faire en sorte que le processeur sélectionne d'autres formes d'ablation par ultrasons à partir de la bibliothèque de formes d'ablation par ultrasons sur la base au moins en partie de la seconde image, les autres formes d'ablation par ultrasons étant sélectionnées et modifiées en comparant les lésions d'ablation observées à partir de la seconde image avec les lésions d'ablation estimées ; et
faire en sorte que les moyens d'ablation par ultrasons opèrent une seconde ablation par ultrasons sur la base au moins en partie des autres formes d'ablation par ultrasons sélectionnées et modifiées.

2. Support de stockage lisible par ordinateur selon la revendication 1, comprenant en outre un code exécutable par ordinateur pour faire en sorte que le système affiche la première image, détermine un plan de traitement en utilisant des formes d'ablation par ultrasons sélectionnées à partir de la bibliothèque, et affiche le plan de traitement, dans lequel le plan de traitement comprend une représentation graphique du volume cible planifié, des points focaux d'administration d'énergie, des formes d'ablation par ultrasons et une ablation composite comprenant de multiples formes d'ablation superposées sur le volume cible planifié à l'intérieur de la région d'intérêt.

3. Support de stockage lisible par ordinateur selon la revendication 1, comprenant en outre un code exécutable par ordinateur pour faire en sorte que le système stocke les autres formes d'ablation par ultrasons sélectionnées et modifiées dans la bibliothèque de formes d'ablation par ultrasons.

4. Support de stockage lisible par ordinateur selon la revendication 1, comprenant en outre un code exécutable par ordinateur pour faire en sorte que le système reçoive les objectifs de traitement entrés par un utilisateur, et sélectionne les formes d'ablation par ultrasons à partir de la bibliothèque de formes d'ablation par ultrasons sur la base au moins en partie des objectifs de traitement et des caractéristiques ultrasonores associées au transducteur ultrasonore (170) qui opère la première ablation par ultrasons.

5. Support de stockage lisible par ordinateur selon la revendication 1, comprenant en outre un code exécutable par ordinateur pour faire en sorte que le système utilise un groupe des formes d'ablation par ultrasons à partir de la bibliothèque de formes d'ablation par ultrasons sur la base au moins en partie d'un modèle d'ablation et de caractéristiques ultrasonores associées au transducteur ultrasonore (170) qui opère la première ablation par ultrasons.

6. Système d'ablation par ultrasons comprenant :
un dispositif d'imagerie (50) pour capturer une première image d'une région d'intérêt ;
un processeur (75) ayant un outil de planification (76) configuré pour déterminer un plan de traitement, le processeur étant configuré pour permettre une délimitation graphique d'un volume cible planifié en utilisant la première image ;
une bibliothèque de formes d'ablation par ultrasons candidates disponibles, une forme d'ablation par ultrasons de la bibliothèque correspondant à une forme de lésion à un point focal respectif, et les formes d'ablation par ultrasons de la bibliothèque ayant été pré-calculées par un outil de modèle acoustique thermique et/ou obtenues par le biais d' expériences ; et
un transducteur ultrasonore (170) configuré pour opérer une première ablation par ultrasons sur le volume cible planifié, dans lequel le processeur est configuré pour choisir des formes d'ablation par ultrasons à partir de la bibliothèque de formes d'ablation, et dans lequel le transducteur ultrasonore est configuré pour appliquer de l'énergie au volume cible planifié sur la base au moins en partie du plan de traitement incluant les formes d'ablation par ultrasons sélectionnées, dans lequel le dispositif d'imagerie est configuré pour capturer une seconde image de la région d'intérêt après application de la première ablation par ultrasons, dans lequel le processeur est configuré pour sélectionner d'autres formes d'ablation par ultrasons à partir de la bibliothèque de formes d'ablation par ultrasons sur la base au moins en partie de la seconde image, et dans lequel le processeur est configuré pour sélectionner et modifier les autres formes d'ablation par ultrasons en comparant les lésions d'ablation observées de la seconde image aux lésions d'ablation estimées.

7. Système selon la revendication 6, dans lequel le transducteur ultrasonore (170) est configuré pour effectuer une seconde ablation par ultrasons sur la base au moins en partie des autres formes d'ablation par ultrasons sélectionnées.

8. Système selon la revendication 7, comprenant en outre un dispositif d'affichage (80) pour présenter la première image et un plan de traitement, dans lequel le plan de traitement comprend une représentation graphique du volume cible planifié, les points focaux d'administration d'énergie, les formes d'ablation par ultrasons et une ablation composite comprenant de multiples formes d'ablation superposées sur le volume cible planifié à l'intérieur de la région d'intérêt.

9. Système selon la revendication 6, dans lequel le processeur (75) est configuré pour recevoir les objectifs de traitement entrés par un utilisateur, et dans lequel l'outil de planification (76) est configuré pour utiliser un ensemble des formes d'ablation par ultrasons provenant de la bibliothèque de formes d'ablation par ultrasons sur la base au moins en partie des objectifs de traitement et des caractéristiques ultrasonores associées au transducteur ultrasonore (170) qui opère la première ablation par ultrasons.

10. Système selon la revendication 6, dans lequel le processeur (75) est configuré pour sélectionner et modifier les autres formes d'ablation par ultrasons en comparant les lésions d'ablation observées de la seconde image aux lésions d'ablation estimées, dans lequel la modification est basée sur au moins l'un de la forme, la taille, l'emplacement et le nombre des formes d'ablation par ultrasons.

11. Système selon la revendication 6, dans lequel le dispositif d'imagerie (50) est configuré pour effectuer au moins une de la seconde imagerie en capturant des mesures de température de la région d'intérêt au moyen d'une imagerie par résonnance magnétique ; d'une imagerie par ultrasons associée à une forme de la région d'intérêt et d'une élastographie.

12. Support de stockage lisible par ordinateur selon la revendication 1, comprenant en outre un code exécutable par ordinateur pour calculer, sur la base du degré de conformité de la forme d'ablation observée avec la forme prévue, un ensemble d'emplacements d'ablation avec les formes d'ablation par ultrasons calculées pour ablater la partie restante du volume de traitement.

13. Système selon la revendication 6, dans lequel le processeur est configuré pour calculer, sur la base du degré de conformité de la forme d'ablation observée avec la forme prévue, un ensemble d'emplacements d'ablation associés aux formes d'ablation par ultrasons calculées pour ablater la partie restante du volume de traitement.
